# EUROPEAN PATENT APPLICATION

(11) **EP 4 403 621 A1**
(43) Date of publication of application: **24.07.2024**
(21) Application number: 23152532.0
(22) Date of filing: 19.01.2023
(51) Int. Cl.: C12M 1/42, C12M 3/00, C12M 1/32, C12M 1/00, C12M 1/34, C12N 5/077

(54) **DEVICE FOR AND USE OF THE DEVICE IN CULTIVATING TISSUE AND OBSERVING CONTRACTIONS OF THE TISSUE**

(71) Applicant: Georg-August-Universität Göttingen Stiftung Öffentlichen Rechts, 37073 Göttingen (DE)
(72) Inventor: Betz, Timo, 37073 Göttingen (DE); Münker, Till, 37073 Göttingen (DE); Hofemeier, Arne, 37124 Rosdorf (DE)
(74) Representative: REHBERG HÜPPE + PARTNER

(57) **Abstract**

A device (1) for cultivating tissue (28) and observing contractions of the tissue (28) comprises a lower component (2), the lower component (2) defining a culture chamber (5) having a transparent bottom, a side wall and an open top, and an upper component (6) fitting on the lower component (2) and having a base plate (7) and two elastic posts (9). Each of the two elastic posts (9) extends downwards from the base plate (7) and has an upper post end (11) that is fixed to the base plate (7) and a free lower post end (10). The two elastic posts (9) are arranged at a lateral post distance (16). When the upper component (6) is fitted on the lower component (2), the two elastic posts (9) extend into the culture chamber (5), the two elastic posts (9) being arranged at lateral wall distances to the sidewall and their lower post ends (10) facing the bottom of the culture chamber (5). Each of the two elastic posts (9) includes a light guide (12) configured and arranged for coupling-in light (29) at the respective upper post end (11) and for coupling-out the light (29) at the respective lower post end (10) and through the bottom of the culture chamber (5). A piezoelectric bending transducer (13) extends downwards from the base plate (7) and has an upper transducer end (14) that is fixed to the base plate (7) and a lower transducer end (15) that is attached to one of the two elastic posts (9) at a vertical distance to its lower post end (10).

## Description

### TECHNICAL FIELD OF THE INVENTION

The present invention relates to a device for cultivating tissue and observing contractions of the tissue. More particular, the present invention relates to a device comprising the features of the preamble of claim 1.

Further, the present invention relates the use of certain embodiments of such a device in cultivating tissue and observing contractions of the tissue.

### PRIOR ART

WO 2014 / 154 704 A1 discloses a method and an apparatus for measuring a contraction characteristic of engineered heart tissue constructs. An engineered heart tissue construct is attached to a separate first support element which comprises or is mechanically coupled to a piezoelectric element being the sensing element of a piezoelectric transducer, and to a second support element spaced from the first support element. Upon contraction of the engineered heart tissue construct, a load is transferred to the piezoelectric element, and the piezoelectric transducer generates and outputs an electrical sensor signal characteristic of the load applied to the first support element by the engineered heart tissue construct due to the contraction thereof. The sensor signal output by the piezoelectric transducer is analyzed, and a contraction characteristic of the engineered heart tissue construct is derived on the basis of the analysis. The first and second support elements are attached to and extend from a base element. The base element is arranged such that each of the first and second support elements extends downwardly from the base element so that the engineered heart tissue construct is suspended on and between the first and second support elements. The base element is arranged on the surface of a multi-well plate such that the base element is supported on the surface, and the engineered heart tissue construct is disposed in a separate one of the wells of the multi-well plate. The second support element may be rigid so that the engineered heart tissue construct carries out an isometric contraction on the first and second support elements. Alternatively, the second support element may be flexible so that the engineered heart tissue construct carries out an auxotonic contraction on the first and second support elements. In an another embodiment at least one of the first and second support elements can be selectively configured to assume either a rigid state or a flexible state, and this at least one support element is changed between its rigid and flexible state for measuring both isometric and auxotonic contractions of the engineered heart tissue construct. Actually, the first support element comprises two strip shaped piezoelectric elements sandwiching between them an intermediate layer stabilizing the piezoelectric elements and providing support for electrical conduits and circuitry operatively coupled to the piezoelectric element. For protecting the piezoelectric elements against external influences, an entire elongate section of the first support element or at least the piezoelectric elements are provided with a protective coating. The piezoelectric elements are electrically connected in series in order to provide together a signal which is twice a signal of a single piezoelectric element. The second support element comprises a straight elongate silicon tube of circular cross-section and a cylindrical elongate rod which is rigid and also of circular cross-section. The rod may be inserted into the tube so that the second support element is rigid, or removed from the tube so that the second support element is flexible. Whereas this apparatus enables for registering loads applied to the first support element for measuring both isometric and auxotonic contractions, the contractions themselves are not monitored.

WO 2016 / 036 532 A1 discloses a bioreactor system configured to provide length control of engineered tissue and to measure properties of the engineered tissue. The bioreactor system comprises a housing including a culture well, a first flexible post suspended in the culture well, and a second rigid post suspended in the culture well, the second post being space from the first post such that the engineered tissue is grown there between. The bioreactor system further comprises a first device for measuring a contractile force of the engineered tissue. The device includes the first post which comprises a fiber-optic that is operatively coupled to a light source and configured to flex when the engineered tissue contracts. The first device further includes a sensor for detecting a degree of movement of the at least one first post. The bioreactor system further comprises a second device for controlling the length of the engineered tissue. The second device includes the rigid second post and an actuator that is operatively coupled to the second post for moving the second post in a controlled manner within the culture well so as to cause a change in a distance between the first post and the second post and thereby control the length of the engineered tissue. The housing includes a base which contains the culture well and a removable lid with the second device being fixedly attached to the lid. The first device includes a base which is coupled to the housing to position and suspend at least one post in the culture well. The first device includes a fiber optic LED emitter that is coupled to the fiber-optic of the first post for generating a light spot that contacts the sensor which comprises a position-sensitive detector (PSD). The PSD is configured to measure tissue contractions by measuring the degree of movement of the fiber-optic by measuring a position of the light spot. A floor of the culture well is formed of a material that allows passage of light from the fiber-optic to the position-sensitive detector which is disposed below the culture well. The fiber-optic passes through the lid with an upper end of the fiber-optic proximate the lid representing a more rigid section of the first post with a lower section thereof representing a flexible section that flexes and responds to applied forces due to tissue contractions. The second post comprises a rigid tungsten post. The actuator comprises a linear actuator that is operatively coupled to a post holder to which the second post is coupled. The post holder is operatively coupled to linear slides that under actuation of the linear actuator results in linear movement of the post holder which results in linear movement of the engineered tissue due to one end of the engineered tissue being coupled to the second post. The bioreactor further includes a pair of electrodes disposed within the culture well outside of the first post and the second post for supplying electrical stimulation across a length of the engineered tissue.

A device for cultivating tissue and observing contractions of the tissue comprising the features of the preamble of independent claim 1 and its use are known from WO 2021 / 229 097 A1. A first component of the device comprises a culture chamber formed therein. The culture chamber comprises a bottom, a side wall and an open top. A second component of the device comprises a base and a pair of posts extending from the base. The first component and the second component are sized and configured to be mated with one another such that, when the second component is placed on the first component, the pair of the posts is inserted into the culture chamber. The bottom of the culture chamber comprises a thin glass bottom, e.g. a microscopy grade glass slide. The length of each of the pair of posts is such that, when the second component is placed on the first component, each of the pair of posts extends substantially down to the bottom of the culture chamber. Further, each of the posts has a diameter of 1 mm or less. This allows for observing tissue provided in the at least one culture chamber and registering differences in distance between the pair of posts located in the culture chamber through the bottom of the culture chamber by means of a high numerical aperture objective required for high resolution microscopy. The detection of posts deflection allows for an accurate quantification of forces exerted by the tissue in the culture chamber onto the posts. However, differences in distance between the pair of posts can only be registered for that culture chamber of a plurality of culture chambers of the device which is actually observed through the bottom of the culture chamber by means of the high numerical aperture objective.

### OBJECT OF THE INVENTION

It is the object of the present invention to provide a device for cultivating tissue and observing contractions of the tissue which, despite of a lean overall design and low costs, allows for determining characteristics of engineered tissue constructs under various conditions at high precision.

### SOLUTION

The object of the invention is achieved by the features of independent claim 1. Dependent claims 2 to 9 relate to preferred embodiments of the device. Claims 10 to 15 relate to preferred uses of certain embodiments of the device.

### DESCRIPTION OF THE INVENTION

A device for cultivating tissue and observing contractions of the tissue according to the present invention comprises a lower component and an upper component. The lower component defines at least one culture chamber having a transparent bottom, a side wall and an open top. The upper component fits on the lower component and has a base plate and at least two elastic posts. Each of the at least two elastic posts extends downwards from the base plate and has an upper post end that is fixed to the base plate, and a free lower post end. The at least two elastic posts are arranged at a lateral post distance. When the upper component is fitted on the lower component, the at least two elastic posts extend into the at least one culture chamber. The posts of the at least two elastic posts are arranged at lateral wall distances to the side wall, and their lower post ends face the bottom of the at least one culture chamber. Further, the device according to the present invention comprises a light guide in each of the at least two elastic posts. The light guide extends along the respective elastic post, and it is configured and arranged for coupling-in light at the respective upper post end and for coupling-out the light at the respective lower post end and through the bottom of the at least one culture chamber. Even further, the device according to the present invention comprises at least one piezoelectric bending transducer extending downwards from the base plate and having an upper transducer end that is fixed to the base plate and a lower transducer end that is locally attached to one of the at least two elastic posts at a vertical distance to its lower post end.

Typically, the lower component will define a plurality of culture chambers, each having a transparent bottom, a side wall and an open top. The bottom of each culture chamber may be transparent to such an extent that it qualifies as a microscopy grade sample slide. The upper component will have at least two elastic posts per culture chamber defined by the lower component. The upper component may comprise three or even more posts per each culture chamber defined by the lower component, and the posts of each pair of these three or more elastic posts may be arranged at a lateral post distance. Due to the fact that the posts are arranged both at the lateral post distances and at the lateral wall distances, movement of the lower post ends within the culture chambers is only restricted by the elasticity of the posts and of the attached at least one piezoelectric bending transducer. In that the at least one piezoelectric bending transducer is attached to the respective elastic post at a vertical distance to its lower post end, the piezoelectric bending transducer may be kept out of a culture medium partially filling the respective culture chamber. Further, the piezoelectric bending transducer does not stiffen the entire elastic post to which it is attached. The light guide included in each of the at least two elastic posts may make up the entire respective post or only a part of the respective post. In the later embodiment, the light guide may be an optical fibre enclosed by a tube-shaped element of the respective post or arranged side by side with another element of the respective post.

In the device according to the present invention, forces exerted on the posts by any tissue connected to the posts can be registered by means of the light coupled-out at the lower post end of any of the elastic posts. If the at least one piezoelectric bending transducer is not attached to the respective post, the force can be calculated from a change in position of the light coupled-out at the lower post end and the bending elasticity of the respective post. If the at least one piezoelectric bending transducer is attached to the respective post, its stiffness and its state of operation have to be considered in calculating the force from the change in position.

In the device according to the present invention, the at least one piezoelectric bending transducer may be used for moving the lower post end of the elastic post to which it is attached to a certain position or to keep the lower post end in this certain position. This use of the piezoelectric bending transducer may be controlled by means of the light coupled-out at the respective lower post end. Keeping the lower post end in a certain position with varying forces applied by tissue attached to the posts simulates an infinite stiffness or rigidity of the respective post. More generally, within the limits of the deformability of the piezoelectric bending transducer, the piezoelectric bending transducer may be controlled by means of the light coupled-out at the respective lower post end to simulate any desired stiffness or rigidity of the respective post, i. e. even a negative stiffness pushing the post in the direction of an external pulling force acting upon the post.

Further, the at least one piezoelectric bending transducer may be operated to exert an external pulling force on the tissue attached to the posts. This pulling force can be monitored by monitoring the light coupled out of the lower post end of the respective post or of the other of the at least two posts, and the length to which the tissue is expanded by application of the pulling force can be monitored by means of the light coupled out of the lower post ends of both of the at least two posts.

All at all, the new device is highly versatile. Further, the device according to the present invention is not dependent on monitoring the position of the lower post ends by a microscope. The light coupled out of the lower post ends is a pointer directly indicating the actual position of the respective lower post and with a high number of photons allowing for a determination of the position of the respective lower post end at high precision with rather simple optical sensors. Nevertheless, the transparent bottom of the culture chamber defined by the lower component allows for microscopically imaging the tissue attached to the posts during their contractions at a high spatial resolution.

In the device according to the present invention, the vertical distance at which the at least one piezoelectric bending transducer is attached to the respective elastic post will typically be between one third and two thirds and preferably between 40 % and 60 % of a post length over which the respective elastic post extends downwards from the base plate. This ensures that the part of the respective elastic post extending over the attachment point still has some elasticity. Further, the at least one piezoelectric bending transducer may be arranged on that side of the respective elastic post facing the other of the at least two elastic posts at the lateral post distance. In this way, the piezoelectric bending transducer is advantageously subjected to pressure instead of pull by any pulling force exerted by tissue connected to the posts, inclusive of any reaction of this tissue to an external pulling force applied to this tissue by the piezoelectric bending transducer.

In the device according to the present invention, an elastic post to which the at least one piezoelectric bending transducer is attached - as such - may have another elasticity, particularly a lower elasticity, than an elastic post to which no piezoelectric bending transducer is attached. The elastic post to which the at least one piezoelectric bending transducer is attached will easily be bent by the piezoelectric bending transducer as desired, even if it is rather stiff. On the other hand, the elastic post to which no piezoelectric bending transducer is attached should be quite elastic to discernibly deform even if only small forces are applied by an attached tissue in order to be able to measure these small forces. Different elasticities of the elastic posts can easily be realized by varying their diameter which has an effect on the stiffness of the posts to the power of two.

In the device according to the present invention, optical connections to the light guides and electric connections to the at least one piezoelectric bending transducer are preferably provided from above of the base plate. Thus, these optical and electric connections are shielded from any culture medium in the at least one culture chamber by the base plate. Whereas the electric connections to the at least one piezoelectric bending transducer will as a rule include a mechanical connection, the optical connections may be provided without mechanical connections in that, for example, light is focused or coupled in the upper post ends in another way through air.

A suitable mechanical connection of the elastic posts and the piezoelectric bending transducer is achieved in that the elastic posts and the piezoelectric bending transducer are fitted into vertical holes in the base plate. The elastic posts and the piezoelectric bending transducer may be force fitted and/or glued in the vertical holes. In one embodiment of the device according to the present invention, the base plate is designed as a printed circuit board (PCB) or combined with an additional PCB arranged on top of the base plate, and the piezoelectric bending transducers are soldered to the PCB.

In another embodiment of the device according to the present invention, the base plate and the elastic posts are different areas of a one-part shaped body made of a transparent material. In this way, the elastic posts are designed as the light guides and the base plate may be used as a manifold in which the light is coupled-in and via which the light is coupled in the individual elastic posts designed as the light guides. In this embodiment, the shaped body may be coated with a reflective coating reflecting the light coupled in, except of at the lower post ends and in at least one region configured for coupling the light in the base plate. For example, the one-part shaped body may be cast of the transparent material or milled out of a block of the transparent. The reflective coating may be applied by dipping the one-part shaped body into a suitable coating solution and by later removing the coating from the lower post ends and the at least one region configured for coupling the light in the base plate.

In order to electrically stimulate any tissue attached to the posts, at least one electrode may extend downwards from the base plate, the at least one electrode having an upper electrode end that is fixed to the base plate and a free lower electrode end extending beneath the lower transducer end. This at least one electrode may be flexible. Additionally or alternatively, the at least one electrode may be arranged at a lateral electrode distance to the elastic posts and the at least one piezoelectric bending transducer. When the at least one electrode is flexible, it may be attached to one of the elastic posts or even form part of this elastic post. It is to be understood, that then the elasticity of the electrode has to be taken into account when determining the force exerted onto the respective post from movements of its lower post end. If the elastic posts are electrically conductive or functionalized to be electrically conductive by, for example, coating them with a metal, the elastic posts themselves may be used as electrodes so that there is no need of additional electrodes.

Like the posts and the piezoelectric bending transducer, the electrode may be fitted into a vertical hole in the base plate, and an electric connection to the at least one electrode may be provided from above the base plate.

In a particular embodiment, the device according to the present invention comprises an upper connector unit configured to be arranged on top of the upper component and including at least one light emitter configured and arranged for coupling the light in the light guides at the upper post ends. This upper connector unit may also include electrical connectors for connecting the at least one piezoelectric bending transducer and any electrode. The at least one light emitter may be a LED or a laser diode. This particular embodiment of the device according to the invention may further comprise a lower connector unit configured to be arranged beneath the lower component and including at least one optical position sensor configured and arranged for registering light positions of the light coupled out of the light guides at the lower post ends and through the bottom of the at least one culture chamber. Preferably, the lower connector unit comprises at least two position sensitive devices (PSDs) or segmented photo diodes, i.e. one optical position sensor per lower post end whose position is to be monitored by registering the light position of the light coupled out of the respective light guide at the respective low post end and through the bottom of the at least one culture chamber. If the lower component comprises a plurality of culture chambers, the lower connector unit will comprise at least two position sensitive devices per culture chamber.

The device according to the present invention may further comprise a controller connected to the at least one optical position sensor of the lower connector unit and to the at least one piezoelectric bending transducer. This controller may be configured for operating the at least one piezoelectric bending transducer based on the light positions registered by the at least one optical position sensor. This controller may further be connected to the at least one electrode and be configured for operating the at least one electrode.

The device according to the present invention comprising the lower connector unit may be used in cultivating tissue and observing contractions of the tissue in that a lateral force acting upon the at least two elastic posts is deduced from at least one of the light positions registered, and that a present value of the lateral posts distance of the two elastic posts is deduced from the at least two light positions registered. It is to be understood that the lateral force can only be deduced from the at least one of the light positions registered when the elasticity or stiffness of the respective elastic post to which the registered light position belongs is known. This knowledge can be achieved by applying known lateral forces to the elastic post and register the resulting movement of the light position registered. Alternatively or additionally, the elastic posts may be manufactured at a high precision from a highly standardized material so that all elastic posts display a same known elasticity.

In a variant of this use of the device according to the present invention, the at least one piezoelectric bending transducer is operated in response to a change of the lateral post distance of the at least two elastic posts such as to keep that lateral post distance constant. Thus, the contraction of the tissue attached to the posts is monitored under isometric conditions.

If, in response to a change of the at least one of the light positions registered, the piezoelectric bending transducer attached to the respective other one of the at least two elastic posts is operated such as to keep that light positions position constant, an isotonic contraction of the tissue attached to the post is observed. More generally, within the limits of the deformability of the piezoelectric bending transducer, the device according to the present invention may be used to define any conditions between isometric and isotonic for observing the contraction of the tissue attached to the posts.

When the at least one piezoelectric bending transducer is operated in response to a change of the lateral post distance of the at least two elastic posts such as to symmetrically distribute the changed lateral post distance over the at least two elastic posts, any movement of the tissue attached to the posts within the culture chamber and any movement of the lower post ends away from the bottom of the culture chamber are minimized. It is to be understood that a changing lateral post distance can only be symmetrically distributed over the at least two elastic posts without changing the force applied to the posts, if both posts are provided with piezoelectric bending transducers.

If the at least one piezoelectric bending transducer is operated in response to a decrease of the lateral post distance of the at least two elastic posts such as to further decrease or increase the lateral post distance, a contraction of the tissue attached to the post is observed under different auxotonic conditions.

Further, the at least one piezoelectric bending transducer may be operated to apply a periodically varying force in a direction of the lateral post distance of the at least two elastic posts to the elastic post to which the at least one piezoelectric bending transducer is attached. The resulting change of the light position registered for the other elastic post may then be analyzed for its phase and its amplitude with respect to the periodically varying force. This analysis allows for determining viscous and elastic characteristics of the tissue attached to the posts. As a piezoelectric bending transducer may have a large hysteresis, the phase of the periodically varying force applied may be derived from the light position registered for the elastic post to which the piezoelectric bending transducer is attached.

Advantageous developments of the invention result from the claims, the description and the drawings.

The advantages of features and of combinations of a plurality of features mentioned at the beginning of the description only serve as examples and may be used alternatively or cumulatively without the necessity of embodiments according to the invention having to obtain these advantages.

The following applies with respect to the disclosure - not the scope of protection - of the original application and the patent: Further features may be taken from the drawings, in particular from the illustrated designs and the dimensions of a plurality of components with respect to one another as well as from their relative arrangement and their operative connection. The combination of features of different embodiments of the invention or of features of different claims independent of the chosen references of the claims is also possible, and it is motivated herewith. This also relates to features which are illustrated in separate drawings, or which are mentioned when describing them. These features may also be combined with features of different claims. Furthermore, it is possible that further embodiments of the invention do not have the features mentioned in the claims which, however, does not apply to the independent claims of the granted patent.

The number of the features mentioned in the claims and in the description is to be understood to cover this exact number and a greater number than the mentioned number without having to explicitly use the adverb "at least". For example, if an upper component is mentioned, this is to be understood such that there is exactly one upper component or there are two upper components or more upper components. Additional features may be added to the features listed in the claims, or these features may be the only features of the respective product.

The reference signs contained in the claims are not limiting the subject matter of the claims. Their sole function is to make the claims easier to understand.

### BRIEF DESCRIPTION OF THE DRAWINGS

In the following, the invention is further explained and described with respect to preferred exemplary embodiments illustrated in the drawings.
- **Fig. 1**: is an exploded perspective view of an embodiment of the device for cultivating tissue and observing contractions of the tissue according to the present invention.
- **Fig. 2**: is an exploded side view of the device according to Fig. 1.
- **Fig. 3**: is an enlarged detail of Fig. 2, the detail being enclosed by a circle A in Fig. 2.
- **Fig. 4**: is a perspective view separately depicting a base plate of the device according to Figs. 1 to 3.
- **Fig. 5**: illustrates the function of the device according to Figs. 1 to 3.
- **Fig. 6 to 10**: illustrate different uses of the device according to Figs. 1 to 3,
- **Fig. 11**: is a diagram showing deflection curves registered in the use according to Fig. 10.

### DESCRIPTION OF THE DRAWINGS

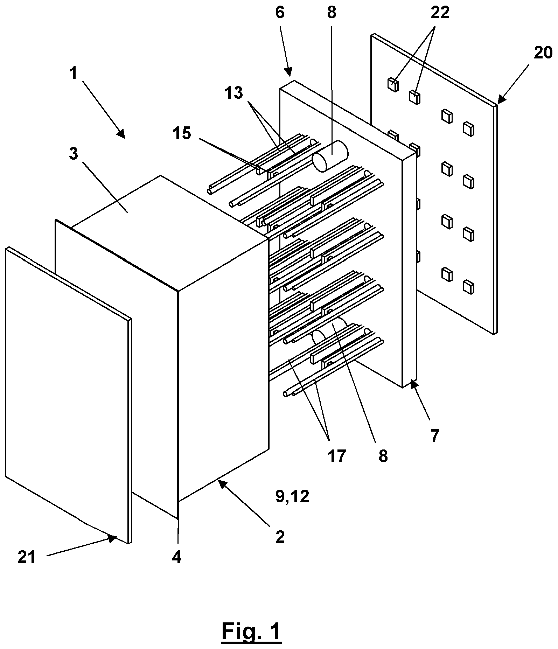

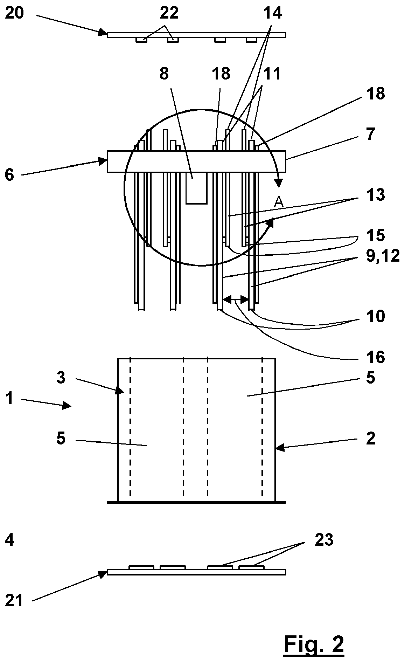

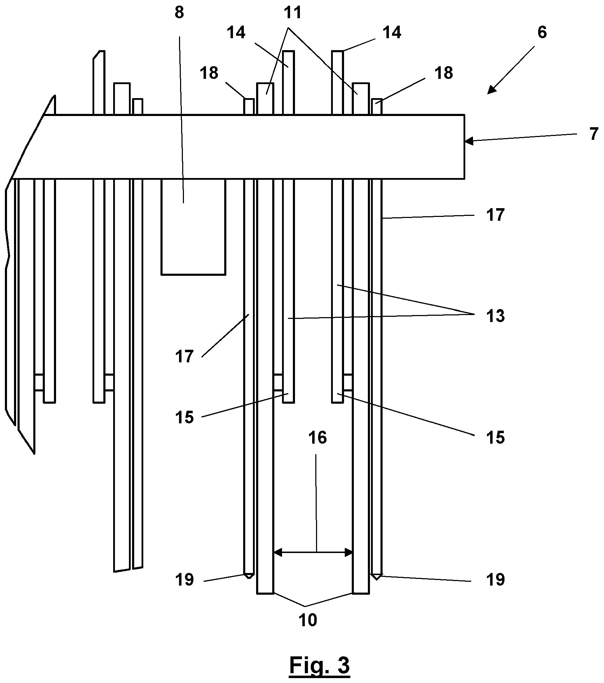

**Fig. 1** and **Fig. 2** show a device 1 for cultivating tissue and observing contractions of the tissue, and **Fig. 3** shows a detail enclosed by a circle A in Fig. 2 at an enlarged scale. The device 1 comprises a lower component 2. The lower component 2 has a block 3 to which a transparent slide 4, which may be a glass slide, is attached. The lower component 2 defines a plurality of eight culture chambers 5 each having a transparent bottom consisting of the transparent slide 4, a side wall provided by the block 3 and an open top. The culture chambers 5 may be of the same design as disclosed in WO 2021 / 229 097 A1.

Further, the device 1 has an upper component 6 to be fitting to the lower component 2. The upper component 6 has a base plate 7. Cylindrical alignment elements 8 extend downwards from the base plate 7 and fit into alignment holes in the block 3 which are not separately depicted here. The alignments elements 8 and the alignment holes allow for fitting the upper component 6 on the lower component 2 such that the open tops of the culture chambers 5 in the lower component 2 are closed by the base plate 7. Per each culture chamber 5, two elastic posts 9 extend downwards from the base plate 7. With the upper component 6 fitted on the lower component 3, these two elastic posts 9 extend into the respective culture chamber 5 such that their lower post ends 10 are close to the transparent slide 4 but do not touch the transparent slide 4. Upper post ends 11 of the elastic posts 9 are fixed to the base plate 7 and extend upwards beyond the base plate 7. Without any external force applied, the elastic posts 9 are straight and parallel with respect to one another. The elastic posts 9 include - or consist of or make up - light guides 12 into which light may be coupled-in at the upper post ends 11. In one embodiment, the light guides 12 are optical fibres. In another embodiment, the light guides are provided by making the elastic posts 9 of a transparent material which is covered with a reflective coating but at the lower and upper post ends 10 and 11, respectively. The light is coupled-out at the lower post ends 10 and through the transparent slide 4, i. e. through the bottom of the respective culture chamber. At about half of the length over which the elastic posts 9 extend downwards from the base plate 7, piezoelectric bending transducers 13 whose upper transducer ends 14 are fixed to the base plate 7 are locally attached to the elastic posts 9 at their lower transducer ends 15. By applying a voltage to the piezoelectric bending transducers 13, the piezoelectric bending transducers 13 are deformed such that they increase a lateral post distance 16 of the two elastic posts 9 arranged within each culture chamber 5. Due to the fact that the piezoelectric bending transducers 13 being arranged between the elastic posts 9, the piezoelectric bending transducers 13 may be bent outwards or increase their length when the voltage is applied in order to increase the lateral post distance 16. On the backs of the elastic posts 9 facing away from the piezoelectric bending transducers 13, electrodes 17 extend downwards from the base plate 7. Upper electrode ends 18 are fixed to the base plate 7, and free lower electrode ends 19 extend beneath the lower transducer ends 15 and end slight above the lower post ends 10. The electrodes 17 are provided for electrically stimulating tissue attached to the elastic posts 9 at their lower post ends 10. The piezoelectric bending transducers 13 are neither intended to get into contact with this tissue nor intended to be immersed in any culture medium within the culture chambers 5.

In order to quantitatively register any deflections of the lower post ends 10 due to forces exerted by tissue attached to the elastic posts 9 or due to operating the piezoelectric bending transducers 13, an upper connector unit 20 and a lower connector unit 21 are provided. The upper connector 20 is to be arranged on top of the upper component 7 and includes one light emitter 22 per light guide 12, i.e. per elastic post 9, here. In another embodiment of the device 1, several or even all optical fibres 12 may be coupled to one light emitter 22. Each light emitter 22 may be a LED or a laser diode, and may comprise an integrated reflector for the light emitted. With the upper connector unit 20 arranged on the upper component 7, the light emitted by each of the light emitters 22 is directed onto the upper post end 11 of the respective elastic post 9 and coupled in its light guide 12. Besides these optical connections to the light guides 12, the upper connector unit 22 will comprise electric connections to the piezoelectric bending transducers 13 and the electrodes 17. However, these electric connections are not depicted here.

The lower connector unit 21 comprises one optical position sensor 23 per elastic post 9, i.e. per light guide 12. The lower connector unit 21 is to be arranged beneath the lower component 2 such that each optical position sensor 23 is located below the lower post end 20 of the respective elastic post 9. Each optical position sensor 23 which may be a PSD or a segmented photo diode registers the light position of incident light coupled out of the respective light guide 12 through the transparent slide 4, i.e. through the bottom of the culture chamber 5. The light position registered by the optical position sensor 23 is indicative of the position of the respective lower post end 10. Thus, the lateral post distance 16 can be derived from the two light positions registered for the respective two elastic posts 9.

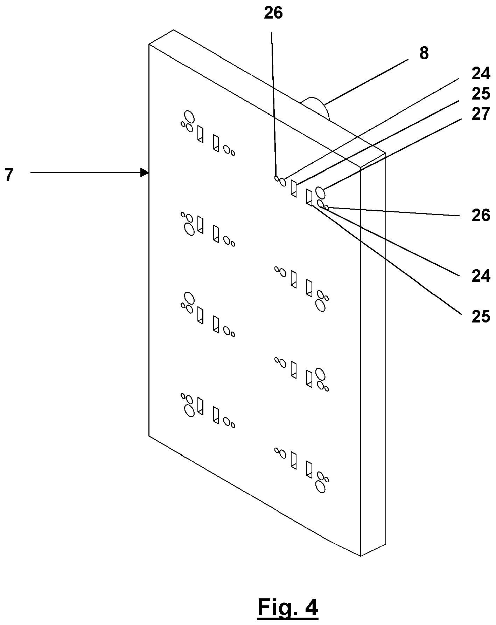

The perspective top view on the base plate 7 according to **Fig. 4** shows one pattern of vertical holes 24 to 27 extending through the base plate 7 per each culture chamber 5 in the lower component 2 according to Figs. 1 and 2. The vertical holes 24 are provided for mounting the two elastic posts 9 to the base plate 7. The vertical holes 25 are provided for mounting the piezoelectric bending transducers 13 to the base plate 7, and the vertical holes 26 are provided for mounting the electrodes 17 to the base plate 7. The respective upper ends 11, 14, 18 of the elastic posts 9, of the piezoelectric bending transducers 13 and of the electrodes 17 are fitted and glued into the vertical holes 24 to 26. The vertical holes 24 to 26 are arranged in a straight line along the lateral post distance 16 according to Figs. 1 and 2. The single further vertical hole 27 allows to inject fluids into the respective culture chamber 5 by means of a syringe and a hollow injection needle passing through the vertical hole 27 up into the respective culture chamber 5. If only one piezoelectric bending transducer 13 is attached to only one of the elastic posts 9, the respective other of the vertical holes 25 may be left free. The same applies if only one or no electrodes 17 are mounted to the base plate 7 per culture chamber 5.

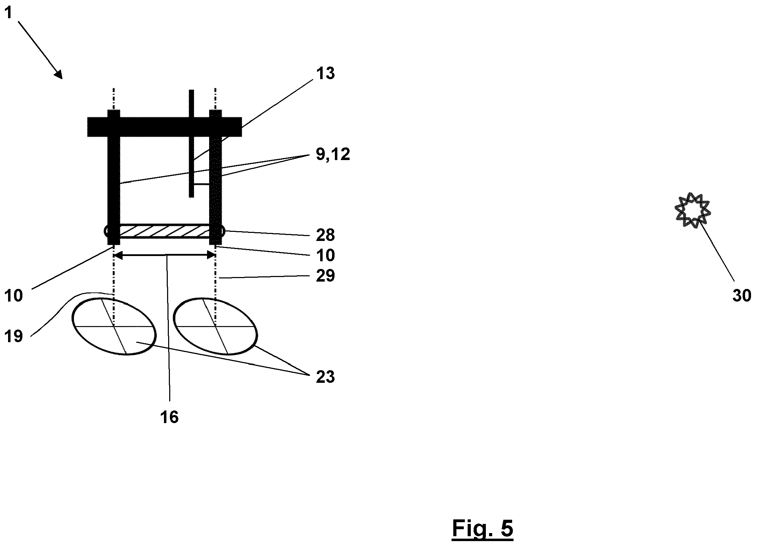

**Fig. 5** illustrates the basic use of the device 1 with respect to the arrangement within one culture chamber 5 according to Fig. 2. The lower component 2, the electrode 17 and the upper component 20 are not depicted. Further, only one piezoelectric bending transducer 13 and only the optical position detectors 23 of the lower connector unit 21 are shown. Additionally, a tissue 28 attached to the elastic posts 9 at their lower post ends 10 is depicted. This tissue may, for example, be an engineered heart tissue or any other contractile tissue. The optical position detectors 23 register the position of light 29 coupled out of the light guides 12 at the lower post ends 10. These light positions change when the tissue 28 contracts, for example due to an external electrical or chemical stimulation 30. This contraction will have an effect on the lateral post distance 16. A change in the lateral post distance 16 can be directly derived from the light positions registered with the optical position sensors 23. Further, the force applied by the tissue 28 to the elastic posts 9 can be derived from the light positions and the bending elasticity of the elastic posts 9.

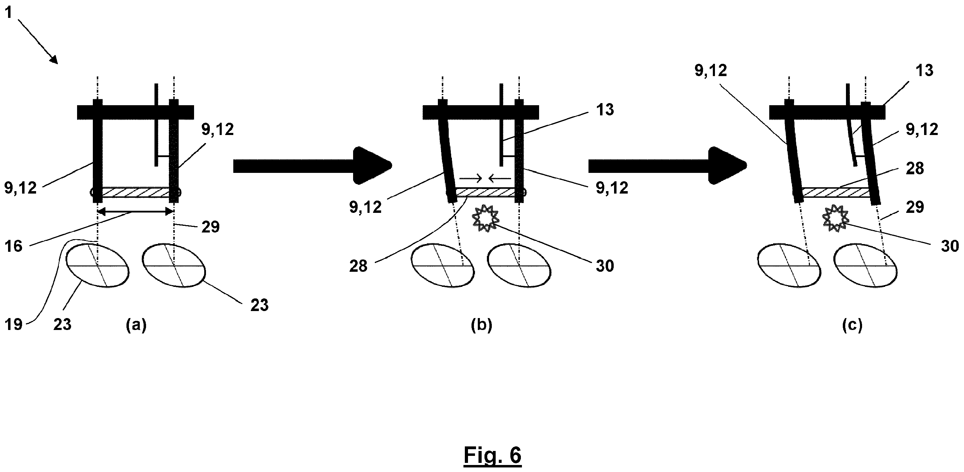

In the use of the device 1 illustrated in **Fig. 6****,** the stimulation 30 results in a contraction of the tissue 28, see Fig. 6(b). By means of operating the piezoelectric bending transducer 13 such as to keep the original lateral post distance 16 constant despite the stimulation 30, see Fig. 6(c), the contraction of the tissue 28 and the resulting forces applied by the tissue 28 to the elastic posts 9 are observed under isometric conditions. In order to keep the lateral post distance 16 constant by means of operating the piezoelectric bending transducers 13 the positions of the light 29 on the optical positions sensors 23 are continuously monitored.

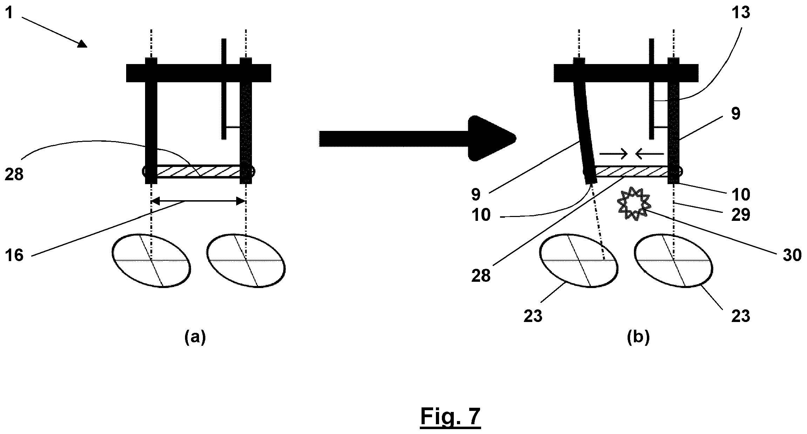

In the use of the device 1 depicted in **Fig. 7****,** the reduction of the lateral post distance 16 due to the stimulation 30 and the resulting contraction of the tissue 28 is accepted. Here, the position of the light 29 coupled out of the lower post end 10 of the elastic post 9 not provided with the piezoelectric bending transducer 13 may be kept constant by operating the piezoelectric bending transducer 13. In this way, the contractions of the tissue 28 are observed under isotonic conditions. The lateral post distance 16 is be derived from the positions of the light 29 coupled out of the lower post ends 10 of both elastic posts 9. The force actually applied by the tissue 28 to the elastic posts 9 can be most easily derived from the position of the light 29 coupled out of the lower post end 10 of the elastic post 9 without piezoelectric bending transducer 13. However, it can also be derived from the position of the light 29 coupled out of the lower post end 10 of the elastic post 9 to which the piezoelectric bending transducer 13 is attached. In any case, the bending elasticity of the respective elastic post 9 has to be taken into account.

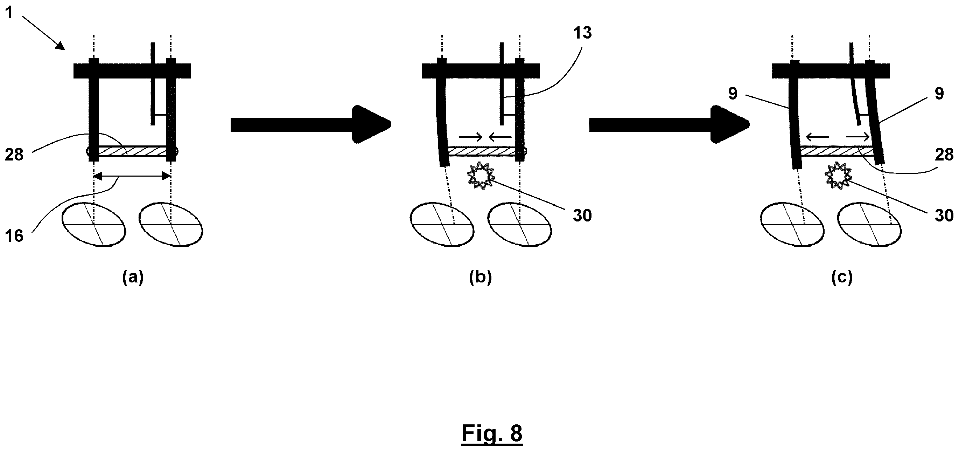

In the use of the device 1 illustrated in **Fig. 8****,** an initial reduction of the lateral post distance 16 due to the stimulation 30 according to Fig. 8(b) is used to operate the piezoelectric bending transducer 13 such as to increase the lateral post distance 16 beyond the original post distance 16 depicted in Fig. 8(a), see Fig. 8 (c). The increase of the lateral post distance 16 may be determined by the force applied by the tissue 28 to the elastic posts 9 such that the tissue 28 is externally stretched in reaction to the tissue 28 applying a pulling force to the posts 9. In this way, the contractions of the tissue 28 are observed under certain auxotonic conditions.

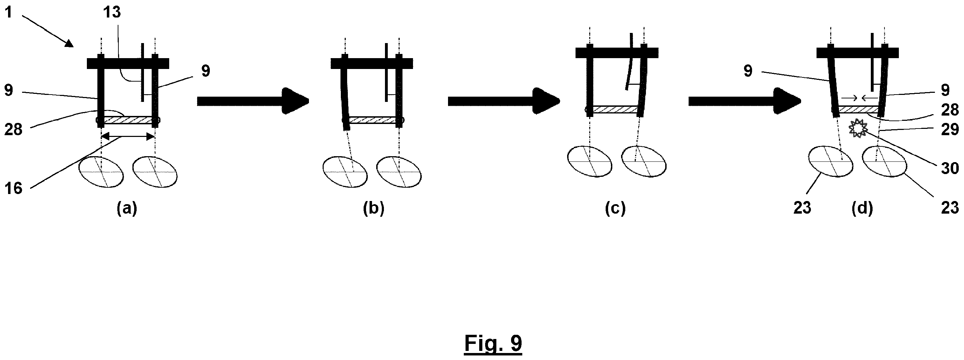

In the use of the device 1 illustrated in **Fig. 9****,** the tissue generates a mechanical pre-tension during its development, which reduces the lateral post distance 16, see Fig. 9(b). This mechanical pre-tension or force applied by the tissue 28 to the posts 9 may then purposefully be reduced by operating the piezoelectric bending transducer 13, see Fig. 9(c). Alternatively, the mechanical pre-tension may be increased by operating the piezoelectric bending transducer 13. However, this alternative is not depicted here. After the desired adjustment of the mechanical pre-tension, the stimulation 30 may be applied and the resulting force exerted by the tissue 28 on the elastic posts 9 and the lateral post distance 19 are derived from the positions of the light 29 on the optical position sensors 23. This use allows for determining contractile forces dependent on mechanical pre-tensions.

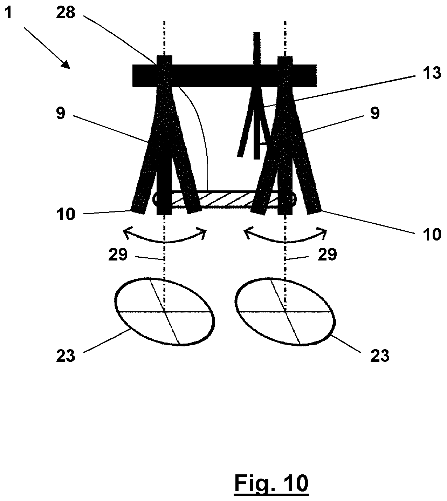

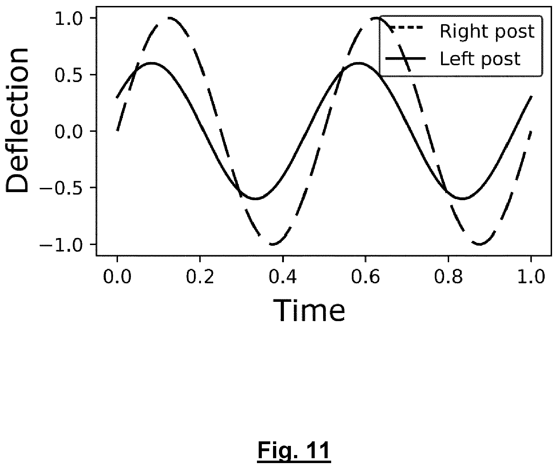

**Fig. 10** illustrates the use of the device 1 for a rheology measurement. The piezoelectric bending transducer 13 is periodically operated to periodically deflect the lower post end 10 of the elastic post 9 to which it is attached. This also results in a periodical deflection of the lower post end 10 of the other elastic post 9 but at a phase offset and at another amplitude. The deflections of both lower post ends 10 are monitored by the positions of the light 29 on the optical position sensors 23. **Fig.11** shows the time courses of the deflections of the right post 9 with the piezoelectric bending transducer 13 with a dashed line and of the resulting deflections of the left post 9 without piezoelectric bending transducer with a continuous line. The differences in phase and amplitude of the two deflections allow for calculating viscoelastic material properties of the tissue 28 attached to the posts 9. The registered deflections include information on the transient lengths and tensions of the tissue 28.

In an actual embodiment of the device 1 of the present invention a post length over which the posts 9 extend downwards from the base plate 7 is in a range from 10 mm to 30 mm. A post diameter is in a range from 0,1 mm to 2 mm, and the lateral post distance 16 of the posts 9 without forces applied to them is in a range from 1 mm to 10 mm.

### LIST OF REFERENCE NUMERALS

- 1: device
- 2: lower component
- 3: block
- 4: transparent slide
- 5: culture chamber
- 6: upper component
- 7: base plate
- 8: alignment element
- 9: elastic post
- 10: lower post end
- 11: upper post end
- 12: light guide
- 13: piezoelectric bending transducer
- 14: upper transducer end
- 15: lower transducer end
- 16: lateral post distance
- 17: electrode
- 18: upper electrode end
- 19: lower electrode end
- 20: upper connector unit
- 21: lower connector unit
- 22: light emitter
- 23: optical position sensor
- 24: vertical hole
- 25: vertical hole
- 26: vertical hole
- 27: vertical hole
- 28: tissue
- 29: light
- 30: stimulation

## Claims

1. A device (1) for cultivating tissue (28) and observing contractions of the tissue (28), the device (1) comprising
- a lower component (2), the lower component (2) defining at least one culture chamber (5) having a transparent bottom, a side wall and an open top, and
- an upper component (6) fitting on the lower component (2) and having a base plate (7) and at least two elastic posts (9), each of the at least two elastic posts (9) extending downwards from the base plate (7) and having an upper post end (11) that is fixed to the base plate (7) and a free lower post end (10), the at least two elastic posts (9) being arranged at a lateral post distance (16),
- wherein, when the upper component (6) is fitted on the lower component (2), the at least two elastic posts (9) extend into the at least one culture chamber (5), the at least two elastic posts (9) being arranged at lateral wall distances to the sidewall and their lower post ends (10) facing the bottom of the at least one culture chamber (5),
**characterized by**
- a light guide (12) included in each of the at least two elastic posts (9), wherein the light guide (12) is configured and arranged for coupling-in light (29) at the respective upper post end (11) and for coupling-out the light (29) at the respective lower post end (10) and through the bottom of the at least one culture chamber (5), and
- at least one piezoelectric bending transducer (13) extending downwards from the base plate (7) and having an upper transducer end (14) that is fixed to the base plate (7) and a lower transducer end (15) that is attached to one of the at least two elastic posts (9) at a vertical distance to its lower post end (10).

2. The device (1) of claim 1, **characterized in**
- **that** the vertical distance at which the at least one piezoelectric bending transducer (13) is attached to the one of the at least two elastic posts (9) is between one third and two thirds, preferably between 40 % and 60 %, of a post length over which the one of the at least two elastic posts (9) extends downwards from the base plate (7), and/or
- **that** the at least one piezoelectric bending transducer (13) is arranged on that side of the one of the at least two elastic posts (9) facing the other of the at least two elastic posts (9) at the lateral post distance (16).

3. The device (1) of claim 1 or 2, **characterized in**
- **that** optical connections to the light guides (12) and electric connections to the at least one piezoelectric bending transducer (13) are provided from above of the base plate (7), and/or
- **that** the at least two elastic posts (9) and the at least one piezoelectric bending transducer (13) are fitted into vertical holes (24, 25) in the base plate (7).

4. The device (1) of claim 1 or 2, **characterized in that** the base plate (7) and the elastic posts (9) are different areas of a one-part shaped body made of a transparent material, wherein the shaped body is optionally coated with a reflective coating except of at the lower post ends and in at least one region configured for coupling-in the light in the base plate (7).

5. The device (1) of any of the preceding claims, **characterized by** at least one electrode (17) extending downwards from the base plate (7) and having an upper electrode end (18) that is fixed to the base plate (7) and a free lower electrode end (19) extending beneath the lower transducer end (15), the at least one electrode (17) being flexible and/or arranged at lateral electrode distances to the at least two elastic posts (9) and the at least one piezoelectric bending transducer (13).

6. The device (1) of claim 5, **characterized in**
- **that** the at least one electrode (17) is fitted into a vertical hole (26) in the base plate (7), and/or
- **that** an electric connection to the at least one electrode (17) is provided from above of the base plate (7).

7. The device (1) of any of the preceding claims, **characterized by** an upper connector unit configured to be arranged on top of the upper component (6) and including at least one light emitter (22), preferably a LED or a laser diode, configured and arranged for coupling the light (29) in the light guides (12) at the upper post ends (11).

8. The device (1) of any of the preceding claims, **characterized by** a lower connector unit configured to be arranged beneath the lower component (2) and including at least one optical position sensor (23), preferably at least two position sensitive devices (PSDs) or segmented photodiodes, configured and arranged for registering light positions of the light (29) coupled out of the light guides (12) at the lower post ends (10) and through the bottom of the at least one culture chamber (5).

9. The device (1) of claim 8, **characterized by** a controller connected to the at least one optical position sensor (23) and to the at least one piezoelectric bending transducer (13) and configured for operating the at least one piezoelectric bending transducer (13) based on the light positions registered, wherein the controller is optionally further connected to the at least one electrode (17) and configured for operating the at least one electrode (17).

10. A use of the device (1) of any of the claims 8 and 9 in cultivating tissue (28) and observing contractions of the tissue (28), **characterized in**
- **that** a lateral force acting upon the at least two elastic posts (9) is deduced from at least one of the light positions registered, and
- **that** a present value of the lateral post distance (16) of the at least two elastic posts (9) is deduced from the light positions registered.

11. The use of claim 10, **characterized in that**, in response to a change of the lateral post distance (16) of the at least two elastic posts (9), the at least one piezoelectric bending transducer (13) is operated such as to keep that lateral post distance (16) constant.

12. The use of claim 10, **characterized in that**, in response to a change of the at least one of the light positions registered, the at least one piezoelectric bending transducer (13) attached to the respective other one of the at least two elastic posts (9) is operated such as to keep that light positions position constant.

13. The use of claim 10, **characterized in that**, in response to a change of the lateral post distance (16) of the at least two elastic posts (9), the at least one piezoelectric bending transducer (13) is operated such as to symmetrically distribute that changed lateral post distance (16) over the at least two elastic posts (9).

14. The use of claim 10, **characterized in that**, in response to a decrease of the lateral post distance (16) of the at least two elastic posts (9), the at least one piezoelectric bending transducer (13) is operated such as to further decrease or increase the lateral post distance (16).

15. The use of claim 10, **characterized in that** the at least one piezoelectric bending transducer (13) is operated to apply a periodically varying force in a direction of the lateral post distance (16) of the at least two elastic posts (9).
